# EUROPEAN PATENT APPLICATION

(11) **EP 4 154 916 A1**
(43) Date of publication of application: **29.03.2023**
(21) Application number: 20425015.3
(22) Date of filing: 19.05.2020
(51) Int. Cl.: A61L 2/10, A61L 2/28

(54) **AUTOMATED LED UV-C SISTEM AIMED AT THE DISIFECTION OF KEYBOARDS AND MAUSE DEVICES FROM BATTERIAS AND VIRUSES**

(71) Applicant: Scatigna, Paolo, 20134 Milano (IT); K-Tronic S.r.l., 10040 Druento (TO) (IT)
(72) Inventor: Scatigna, Paolo, 20134 Milano (IT); K-Tronic S.r.l., 10040 Druento (TO) (IT)

(57) **Abstract**

The Industrial patent consists of a mobile trolley with LED UV-C LIGHT BAR within. It is motorised and moves on rails adjustable in height. The led lights are placed on the bottom side of the tool making easier to extraction and substitution.

Once placed the keyboard, mouse device or both, under the cart, the sterilisation process will begin.

The artificial optical radiations emitted by the LED UV-C certify that the radiations have optimal margin upon the expected limits regarding the sterilising action on pathogens and the removal of any bacteria, from poliomyelitis to anthrax.

Both the technical and the electronic part, as well as the radiation emitted by the LED UV-C lights, have an appropriate safety margin for the users. The shell - shaped coverign of the LED prevents users from placing their hands underneath them when the device is working, furthemore if the tool is picked up or turned around, the device activity interrupts.

## Description

### TECHNICAL PHASE

This EUROPEAN INDUSTRIAL PATENT is hereby registered at the Ministry of Economic Development - Trademarks and Patents Office, 3 pages, on May 2020, with 3 pages of technical designs, 3 pages of technical report and 12 pages of Scientific Analysis on the artificial optical radiations emitted by the LED UV-C device.

The technical designs of the Patent highlight the ease and fluidity with which the system operates. The four pins at the base of the system are adjustable in hight so that the user may employ the tool with safety and comfort.

The INDUSTRIAL PATENT essentially consists of a mobile trolley containing a LED UV-C light bar within. Said trolley is motorised and moves on rails that are adjustable in height. The trolley has on its case a keyboard with no. 2 keys: the first (ON/OFF) used for power and the second for the selection of one of the sterilisation programmes. A display will show which programme has been selected as well as potential anomalies in the functioning of the Patent. It will also include information regarding battery charge.

The mobile trolley contains a lithium battery that can be recharged through the USB socket of a computer or of any other 5-volt power supply (e.g. 220 watts electricity with a 5-volt transformer).

Through the technical designs one can see that the upper central part (also referred to as mobile trolley) contains a small engine and features on its case a power button with related LED light, the programme selection button and related LED light. On the bottom of the trolley, the LED UV-C lights window contains a series of UV-C lamps that carry out the bacterial sterilisation through a left-to-right movement.

The LED UV-C lights assembly kit has been placed on the bottom side of the tool, making it accessible for extraction and light substitution by removing the top part of the case.

Through the addition of a flexible USB cable the tool can be used in various contexts, from home to the workplace, in order to apply disinfection measures from germs and bacterias.

### ELECTRONIC PHASE

Once placed the keyboard, mouse device, or both under the cart (ensuring a distance between the object and the sterilisation tool between 15 and 25 mm through the adjustable pins) and after having adjusted the trolley's run length through the designated termination point, the desired programme can be selected. Pressing the power button the sterilisation process will begin. Depending on which programme is selected, the time length of the process will vary.

### BACTERIA AND RADIATION IN SCIENTIFIC RESEARCH

The INDUSTRIAL PATENT with LED UV-C light has been created to ensure the sterilisation of productivity tools such as keyboards and mouse devices of any work or personal computers. Attached to the present document are 12 pages of scientific analysis on the artificial optical radiations emitted by the LED UV-C device, which, as curated by the company Nuovi Servizi Ambientali s.r.l. legally headquartered in Corso Re Umberto 12, 10121 Turin, and as signed by Simone Cipriani, certify that the radiations have optimal margins upon the expected limits regarding the sterilising action on pathogens and the removal of any bacteria, from poliomyelitis to anthrax.

### SAFETY SYSTEMS

As described, both the technical and the electronic part as well as the radiation emitted by the LED UV-C lights, have an appropriate safety margin for the users. The Industrial Patent is in fact equipped with two safety systems. The first is in the form of the shell-shaped covering of the LED UV-C lights that prevents users from placing their hands underneath them when the device is working. The second feature automatically interrupts the device's activity if the tool is picked up or turned around, so as to avoid any misuse.

### ADMINISTRATIVE REPORT

The patent is submitted by Mr. Paolo Scatigna (inventor and creator of the industrial patent), born in Taranto on 11/06/1960 fiscal code SCTPLA60H11L049N, residing in Milan in Via San Dionigi 28, 20123, and by Mrs. Milete Angela, chief executive officer of the company K-Tronic s.r.l. (industrial patents engineering firm), p. IVA 03873380012, production of industrial membrane keyboards, Via Alessandro Volta 31, 10040 Druento (TO). She was born in San Martino in Pensilis (CB) on 14/02/1948, fiscal code MLTNGL48B54H990V, and resides in Via Antonio Axerio 1, Druento (TO)

Mr. Paolo Scatigna, full owner of the INDUSTRIAL PATENT, waives a 50% share to the company K-Tronic s.r.l., with the two parties equally owning 100% of the INDUSTRIAL PATENT.

In the event that one of the parties wishes to sell, lease, cede to others through community of property or through any other form, or sell their share to third parties, the party will have to first consider the perpetual right of first refusal in accordance with the law. The ceding party will have to send a proposal of the share's transfer to the other 50% shareholder through registered post with acknowledgment of receipt. In case of refusal by the other 50% shareholder, the ceding party is free to sell its share to third parties through merger by incorporation. The parties will be safeguarded by an Italian notary contract that will be stipulated upon submitting the Patent. The legal clauses described so far as well as the 50% parts are also valid for the parties' heirs.

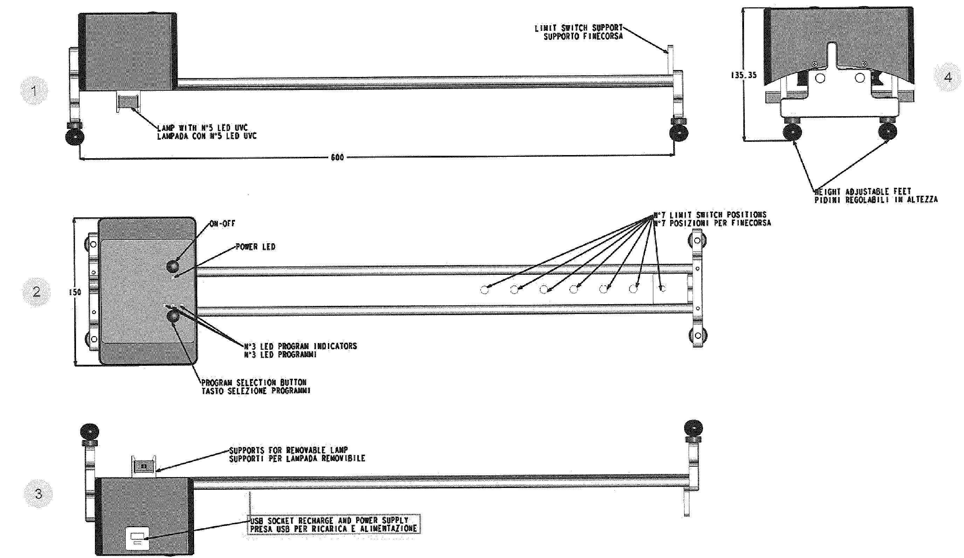

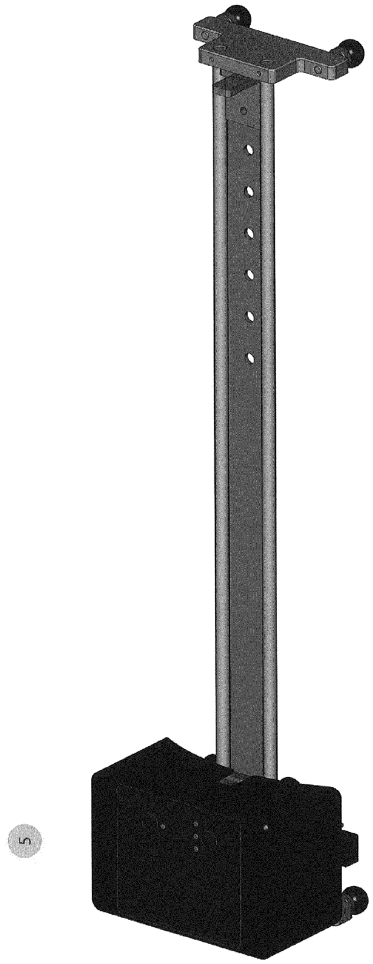

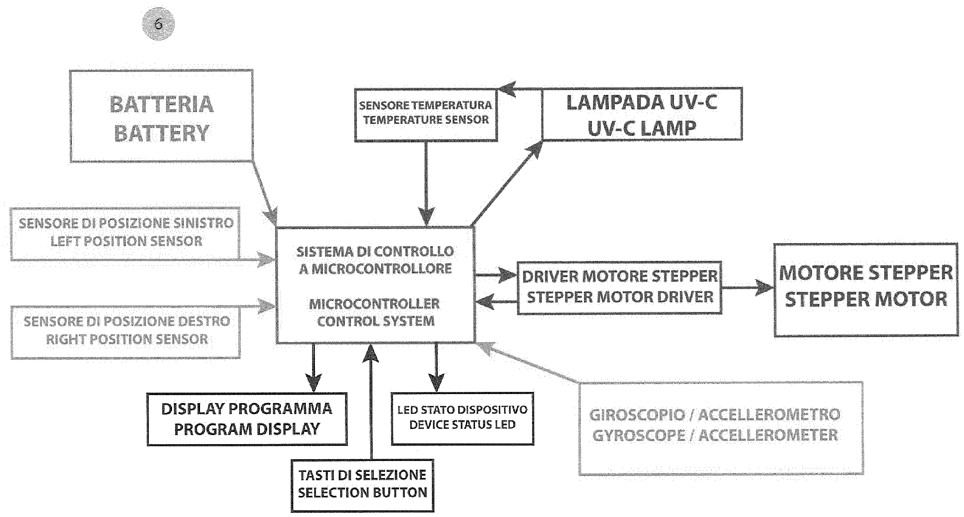

## Claims

1. the method of the automated UV-C LED SYSTEM which sanitizes industrial and personal keyboards and mice from bacterias and viruses, the method comprising:
that the Test carried out, against the activation power of 5 volts, its 5 LEDs at 280 nm with a fixed device used (on the limit of UV-C with UV-B) the occasional time that must be spent to start and automatically shut down the operation of the product, it has been determined that no traces of contamination and/or optical changes remain on humans using the product;
instead, based on seconds and distance used for Viruses, Bacteria, etc. the times of destruction have been effective. Starting from a minimum distance, 0.5 cm., with a minimum of 18.94 seconds, the destruction of Paratyphoid Salmonella Typhimurium is obtained, as also for the destruction of Anthrax Spores, where we have seen a maximum distance of 5, 08cm. against a maximum of 942.86 seconds, all the data are already confirmed and explained in the known technical figure.

2. the method according to claim 1,
Wherein we treated the Test by increasing the LEDs to 280 nm. (from 5 leds to 12 leds remaining on the limit of UV-C with UV-B), the activation power remained at 5 volts, for the occasional time needed to turn the system on and off, lastly, through the device that from fixed can become semi-automatic and movable for the product on and off function, well, it has been ascertained that even here there are no traces of contamination and/or optical variations on human beings;
instead, based on seconds and distance used for Viruses, Bacteria, etc. the times of destruction were much more effective. In fact, starting from a fixed distance of 1 inch (2.54 cm.) the destruction of Paratyphoid Salmonella Typhimurium was seen in 6.100 seconds, as well as for the destruction of Anthrax Spores in 46.200 seconds, instead on the destruction of the Sars virus. Cov. 2 or Covid 19 there was a differential between the axis and the center, i.e., in axis they range from 66.7 to 146.2 seconds while in the center they range from 56.4 to 94.9 seconds, all data are already confirmed in the subsequent known technical representation.
